# EUROPEAN PATENT APPLICATION

(11) **EP 0 582 259 A2**
(43) Date of publication of application: **09.02.1994**
(21) Application number: 93112372.3
(22) Date of filing: 02.08.1993
(51) Int. Cl.: A61K 9/10, A61K 47/32, A61K 47/38

(54) **Preparation for local treatment containing particulates of water soluble polymers in suspension**

(30) Priority: 03.08.1992 JP 206640/92
(71) Applicant: Senju Pharmaceutical Co., Ltd., Osaka-shi, Osaka 541 (JP)
(72) Inventor: Morita, Yasushi, Toyonaka-shi, Osaka (JP); Kimura, Masako, Kakogawa-shi, Hyogo-ken (JP)
(74) Representative: Kinzebach, Werner, Dr.

(57) **Abstract**

Presented is an aqueous suspension preparation for local treatment with an improved bioavailability, which contains; suspension particulates of one or more water soluble polymers which will dissolve or gel at a pH within the pH range of secreted fluid at a local site of the body to which said preparation is intended to be applied; and a drug acceptably applicable to said local site.

## Description

### BACKGROUND OF THE INVENTION

The present invention relates to an aqueous preparation for local treatment characterized in that it comprises suspension particulates of water soluble polymers which will dissolve or gel at a pH within a pH range of secreted fluid at a local site of the body and a drug which can be locally applied. In further detail, the present invention relates to a preparation for local treatment, the drug bioavailability thereof being enhanced by letting the locally applicable drug to coexist with suspension particulates of one ore more water soluble polymers which will dissolve or gel at a pH within the pH range of secreted fluid at a local site of the body, whereby increasing a retaining effect at the applied site and further increasing the viscosity of the preparation through dissolving or gelation of the polymers due to the pH of the secreted fluid at the site.

Treatment using an ointment is one of the choice for local treatment procedures. However, for sites covered with mucous membrane such as the eye, the ear and the nose, for example, ointments are rarely used due to problems such as external irritation. Thus, for these sites, it is presently common to instill a drug in a form of an aqueous solution or suspension directly to the treatment site, i.e., into the eye, the ear or the nose. However, in the case of eye drops, nearly half of the applied solution will overflow out of the lower eyelid directly after its topical application into the eye, and the rest of the solution will flow into the nasal cavity within a short time. As for nasal drops, similarly, since most part of the solution is eliminated into the throat, it is for relatively a short time that an effective concentration of the drug can be retained at the local site. Therefore, the bioavailability of the drug is very low in these treatment procedures, which makes it necessary to increase the frequency of application for some drugs, accordingly.

In the field of ophthalmology, it is known that the intraocular transfer and pharmacological effect of eye drops are enhanced by increasing its kinematic viscosity by an addition of various viscous excipients to the eye drops, and that a drug is well retained in the anterior part of the eye by topically applying the drug in the form of suspension particulates. However, a great increase in bioavailability of drugs has never been attained only by these means.

In recent years, therefore, drug delivery systems for local treatment are under development in order to enhance the bioavailability of drugs. Among them, H-W Hui et al. [International Journal of Pharmaceutics, 26: 203-213(1985)] describe that the transferability of progesterone into the anterior aqueous humor is improved by preparing progesterone in the form of cross-linked acrylic acid polymer microspheres. Moreover, Nisimura et al.[Chemotherapy, 39: 728(1991)] report that dibekacin-containing microspheres may have a possibility to become a new preparation for sustained local treatment for infectious diseases in the field of oto-rhinolaryngology.

The microspheres offer an advantage that they can be easily instilled into the eye, the ear or the nose in the same manner as in the case of general types of suspension eye drops, ear drops or nasal drops. However, in spite that the optimal size of the suspended particulates differs depending on the site of application, it is not easy to prepare microspheres of a desired, uniform particulate size, and, there is a disadvantage that it is difficult to remove remaining solvents used during the microsphere preparation.

Moreover, for encapsulating a drug in microspheres, it is necessary to prepare microspheres so that the expected effect of the drug may be exhibited without diminution and the effect may be sustained. At present, however, as V. Vidmar et al. [Journal of Microencapsulation, 2: 289-292(1985)] report, water soluble drugs may be rapidly eluted from microspheres and there may be a case that microspheres to serve as sustained release preparations only directly after the preparation of the microsphere suspension.

In this situation, the inventor intensely investigated in search of a preparation for local treatment which is more easily producible than microspheres and has a high bioavailability in the site of application.

### SUMMARY OF THE INVENTION

The inventors have found that, when water soluble polymers which will dissolve or gel at a pH within the pH range of secreted fluid from the body are applied in a local site in the form of an aqueous suspension, the water soluble polymers are retained at the applied site as suspension particulates, and then the water soluble polymers will dissolve or gel in the secreted fluid at the local site. Moreover, the inventors have unexpectedly found that, when a drug is applied with coexisting suspension particulates of the water soluble polymers, the nature of these water soluble polymers enhances the retention of drugs at the local site applied, thus providing a new preparation for local treatment that can enhance the bioavailability of a drug. Thus, the present invention is completed.

### DETAILED DISCUSSION

Therefore, the present invention is an aqueous preparation for local treatment characterized in that it comprises:
(a) suspension particulates of one or more water soluble polymers which will dissolve or gel at a pH within the pH range of secreted fluid at a local site of the body, and
(b) a drug which can be locally applied.

The present invention is also a method for enhancing the local bioavailability of a drug contained in an aqueous preparation for local treatment by dispersing in said aqueous preparation particulates of one or more water soluble polymers which will dissolve or gel at a pH within a pH range of secreted fluid at the local site of the body to which said preparation is intended to be applied.

As water soluble polymers to be employed in the present invention, polymers which will dissolve or gel at a pH of the secreted fluid at a local site of the body [e.g. tear (pH 7.0 to 8.5), nasal secretion (pH 5.0 to 8.0, generally considered in the field of oto-rhinolaryngology) or otorrhea (pH 5.0 to 8.0, generally considered in the field of otorhinolaryngology)] to which the preparation of the present invention is intended to be applied can be used. By way of example, polymers which will dissolve or gel at a pH within a range from 5 to 8 can be advantageously used.

As water soluble polymers to be employed in the present invention which will dissolve at a pH within the pH range of secreted fluid at a local site of the body, water soluble polymers which will dissolve or gel only at a pH of not less than e.g. 5, for example esters from dicarboxylic acid and hydroxyalkylcellulose and derivatives thereof, such as hydroxypropylmethylcellulose phthalate and hydroxypropylmethylcellulose acetate succinate and the like, can be preferably used, Further, water soluble polymers which will dissolve or gel only at a pH of not less than e.g. 5, for example acrylic acid resins such as methacrylic acid/acrylic acid ester copolymer and methacrylic acid/methacrylic acid ester copolymer such as those commercially available under the trade names of Eudragit L and Eudragit S, for instance, can be used suitably. These water soluble polymers can be used independently or as a mixture of 2 or more of them.

In the preparation for local treatment of the present invention, the amount to be added of the water soluble polymers which will dissolve or gel at a pH within the pH range of secreted fluid at a local site of the body is suitably selected according to their types and to the drug to be incorporated. Usually, they are added at approximately 0.05 to 30 w/v%, preferably about 0.1 to 10 w/v%.

The mean size of the particulates of the water soluble polymer employed in the present invention which will dissolve or gel at a pH within the pH range of secreted fluid at a local site of the body can be suitably selected according to the method and site of application of the preparation. The mean size is within a range from 0.1 to 400 µm in usual cases, and preferably from 1 to 200 µm.

As for the method for producing the particulates of the water soluble polymers which will dissolve or gel at a pH within the pH range of secreted fluid at a local site of the body to be employed in the preparation for local treatment of the present invention, generally used methods such as grinding method, spray drying method, solvent evaporation method and the like can be suitably utilized. The particulates thus obtained are then dispersed in a liquid by a conventional method.

In the preparation for local treatment of the present invention, it is possible to suitably adjust the length of time for which the preparation will be retained at the local site applied, based on the type, concentration and particulate size of the employed water soluble polymers which will dissolve or gel at a pH within the pH range of secreted fluid at a local site of the body. The selection of such suitable condition can be easily determined based on a simple experiment well known to the person skilled in the art (for example, the method that will be described in "Transferability test" section in this specification).

As for drugs to be incorporated in the preparation for local treatment of the present invention, any drugs (water soluble drugs or scarcely soluble drugs) usually used locally can be advantageously employed and they include, by way of example; anti-allergics such as diphenhydramine hydrochloride, chlorphenyramine maleate, glycyrrhizin, sodium cromoglycate and amlexanox; vasoconstrictors such as naphazoline nitrate, oxymetazoline hydrochloride and phenylephrine hydrochloride; steroidal anti-inflammatories such as fluorometholone, dexamethasone, cortisone and prednizolone; non-steroidal anti-inflammatories such as azulene, indomethacin and pranoprofen; anti-microbials such as cephalosporin, erythromycin, cefmenoxime, chloramphenicol, gentamicin and kanamycin; local anesthetics such as procaine hydrochloride and lidocaine hydrochloride; β-blockers such as timolol maleate, carteolol hydrochloride and bupranolol hydrochloride; miotics such as pilocarpine hydrochloride, distigmine bromide and physostigmine sulfate; mydriatics such as atropine sulfate and phenylephrine hydrochloride; and anti-cataracts such as pirenoxine and glutathione.

Among these drugs, those scarcely soluble drugs can be used after solubilizing with solubilizers or they can be used in suspension. The examples of the solubilizers include surface active agents such as polysorbate 80, water soluble polymers such as polyvinylpyrrolidone and polyvinylalcohol, and complex forming compounds such as cyclodextrins and the like.

In these preparations, additives conventionally used for aqueous preparations can be added. The examples of such additives include preservatives (such as p-hydroxybenzoates , benzalkonium chloride, chlorobutanol), stabilizers (such as sodium edetate, sodium citrate), suspending agents (such as carboxymethylcellulose, hydroxypropylcellulose), isotonizers (sodium chloride, sorbitol, glycerol), surface active agents (such as polysorbate 80) and buffering agents (such as sodium dihydrogen phosphate, boric acid, citric acid), and pH adjusting agents (such as hydrochloric acid, acetic acid, sodium hydroxide).

The preparation for local treatment of the present invention can be produced in the form of, for example, eye drops, ear drops and nasal drops used for treatment of diseases in the fields of ophthalmology and otorhinolaryngology.

The preparation for local treatment of the present invention can improve the retention of a drug in the applied site, thereby enhancing the bioavailability of the drug. In addition, the preparation for local treatment of the present invention can be produced more easily and more economically compared with the case which requires a preparation of microspheres.

The present invention will be described in further detail and its effect will be demonstrated in the following examples and test. However, the examples are only for explanation of the present invention, and therefore they do not limit the scope of the present invention.

### EXAMPLE 1 Eye drops

| | |
|---|---|
| Fluorometholone | 0.1 g |
| Hydroxypropylmethylcellulose acetate succinate | 1.0 g |
| Sodium dihydrogen phosphate | 0.1 g |
| Sodium chloride | 0.9 g |
| Polysorbate 80 | 0.2 g |
| Benzalkonium chloride | 0.005 g |
| Distilled water | to total 100 ml |

In 80 ml of distilled water are dissolved 0.1 g of sodium dihydrogen phosphate, 0.9 g of sodium chloride, 0.2 g of polysorbate 80 and 0.005 g of benzalkonium chloride, and the pH of the solution is adjusted to about 4.5 with 0.1 N hydrochloric acid. Then, 0.1 g of fluorometholone and 1.0 g of hydroxypropylmethylcellulose acetate succinate having a particulate size of about 2 µm are dispersed in the solution, and distilled water is added to make 100 ml to prepare eye drops.

### EXAMPLE 2 Eye drops

| | |
|---|---|
| Fluorometholone | 0.02 g |
| Hydroxypropyl-γ-cyclodextrin | 4.0 g |
| Hydroxypropylmethylcellulose acetate succinate | 1.0 g |
| Glycerol | 2.6 g |
| Polysorbate 80 | 0.2 g |
| Sodium dihydrogen phosphate | 0.1 g |
| Benzalkonium chloride | 0.005 g |
| Distilled water | to total 100 ml |

In 80 ml of distilled water are dissolved 0.02 g of fluorometholone and 4.0 g of hydroxypropyl-γ-cyclodextrin.

In the solution are then dissolved 0.1 g of sodium dihydrogen phosphate, 2.6 g of glycerol, 0.2 g of polysorbate 80 and 0.005 g of benzalkonium chloride, and the pH of the solution is adjusted to about 4.5 with 0.1 N hydrochloric acid. Then, 1.0 g of hydroxypropylmethylcellulose acetate succinate having a particulate size of about 2 µm is dispersed in the solution, and distilled water is added to make 100 ml to prepare eye drops.

### EXAMPLE 3 Otic drops

| | |
|---|---|
| Kanamycin sulfate | 2.0 g |
| Hydroxypropylmethylcellulose phthalate | 5.0 g |
| Sodium dihydrogen phosphate | 0.1 g |
| Sodium chloride | 0.9 g |
| Methyl p-hydroxybenzoate | 0.02 g |
| Propyl p-hydroxybenzoate | 0.01 g |
| Distilled water | to total 100 ml |

In a heated 80 ml of distilled water are dissolved 0.9 g of sodium chloride, 0.02 g of methyl p-hydroxybenzoate and 0.01 g of propyl p-hydroxybenzoate. In the solution after cooling are dissolved 2.0 g of kanamycin sulfate and 0.1 g of sodium dihydrogen phosphate, and the pH of the solution is adjusted to about 4.0 with 0.1 N hydrochloric acid. Then, 5.0 g of hydroxypropylmethylcellulose phthalate having a particulate size of about 10 µm is dispersed in the solution, and distilled water is added to make 100 ml to prepare ear drops.

### EXAMPLE 4 Nasal drops

| | |
|---|---|
| Sodium cromoglycate | 2.0 g |
| Eudragit L | 0.5 g |
| Sodium edetate | 0.01 g |
| Benzalkonium chloride | 0.04 g |
| Sodium dihydrogen phosphate | 0.1 g |
| Distilled water | to total 100 ml |

In 80 ml of distilled water are dissolved 2.0 g of sodium cromoglycate, 0.01 g of sodium edetate, 0.04 g of benzalkonium chloride and 0.1 g of sodium dihydrogen phosphate, and the pH of the solution is adjusted to about 4.5 with 0.1 N hydrochloric acid. Then, 0.5 g of Eudragit L having a particulate size of about 1 µm is dispersed in the solution, and distilled water is added to make 100 ml to prepare nasal drops.

### TRANSFERABILITY TEST OF FLUOROMETHOLONE EYE DROPS TO CONJUNCTIVAL TISSUE

As demonstrated in the transferability test below, when hydroxypropylmethylcellulose acetate succinate particulates are suspended in eye drops, the retention of the drug in the anterior part of the eye is improved and the bioavailability of fluorometholone is increased.

### Test method:

The fluorometholone eye drops prepared in Example 1 was used as "eye drops A". 50 µl of the eye drops A was instilled into rabbit eyes, and the time course of the fluorometholone concentration in the conjunctiva was assayed using high performance liquid chromatography. As a control, ODOMEL® 0.1 % ophthalmic suspension (0.1 w/v% fluorometholone, Senju Pharmaceutical Co., Ltd.) was used.

### Test result:

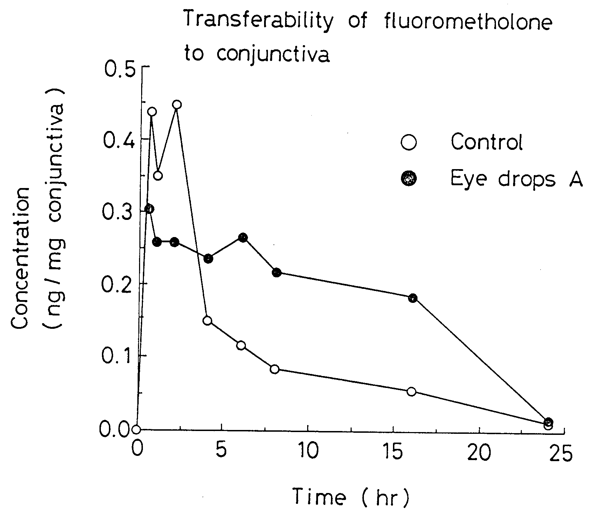

The figure shows the concentration-time profile curves of fluorometholone in the rabbit conjunctiva after the instillation of eye drops A ( ● ) and control fluorometholone eye drops ( ○ ), respectively. The axis of abscissas indicates the time (hr) after the instillation, and the axis of ordinate indicates the amount (ng) of fluorometholone in 1.0 mg of the conjunctival tissue. Each point on the curves represents the the mean value (n=4). AUC (area under the curve) for each of the eye drops was calculated from the concentration-time profile curves for the control and the eye drops A. In the control, the concentration of fluorometholone in the conjunctiva reached its maximum 2 hours after the instillation and rapidly disappeared thereafter. In the eye drops A, although the maximal concentration of fluorometholone in the conjunctiva was lower than that of the control, the fluorometholone concentration in the conjunctival tissue (1 mg) up to 16 hours after the instillation was retained at about 0.184 ng, and its AUC was 1.7 times as much as that of the control.

## Claims

1. An aqueous suspension preparation for local treatment containing;
suspension particulates of one or more water soluble polymers which will dissolve or gel at a pH within the pH range of secreted fluid at a local site of the body to which said preparation is intended to be applied, and
a drug acceptably applicable to said local sites.

2. A preparation according to claim 1, wherein said one or more water soluble polymers are selected from a group consisting of esters from dicarboxylic acid and hydroxyalkylcellulose and derivatives thereof and acrylic acid resins.

3. A preparation according to claim 2, wherein said hydroxyalkylcellulose is selected from a group consisting of hydroxypropylmethylcellulose phthalate and hydroxypropylmethylcellulose acetate succinate.

4. A preparation according to claim 2, wherein said acrylic acid resins are selected from a group consisting of methacrylic acid/acrylic acid ester copolymer and methacrylic acid/methacrylic acid ester copolymer.

5. A preparation according to claim 1, 2, 3 or 4, wherein said preparation is in the form of eye drops, ear drops or nasal drops.

6. A method for enhancing the local bioavailability of a drug contained in an aqueous preparation for local treatment which comprises dispersing, in said aqueous preparation, particulates of one or more water soluble polymers which will dissolve or gel at a pH within a pH range of secreted fluid at the local site of the body to which said preparation is intended to be applied.
